# EUROPEAN PATENT APPLICATION

(11) **EP 2 704 047 A2**
(43) Date of publication of application: **05.03.2014**
(21) Application number: 13181977.3
(22) Date of filing: 28.08.2013
(51) Int. Cl.: G06F 19/00

(54) **Remote diagnostic system and method for medical image**

(30) Priority: 30.08.2012 JP 2012190378
(71) Applicant: Fujifilm Corporation, Minato-ku Tokyo (JP)
(72) Inventor: Ohta, Yasunori, Kanagawa (JP)
(74) Representative: Vierheilig, Achim

(57) **Abstract**

In a remote diagnostic system, one remote service provider (58a) among plural remote service providers (12-15, 38a-38n, 58b) is requested by a health care provider (service requester) to diagnose a medical image (59b), and the image is transmitted thereto. Attribute information (57) constituted by at least one of an object region and symptom information (42b, 57b) of an object in the image is input for the request of diagnosis. The one remote service provider is selected among the plural remote service providers, to transmit the image and the attribute information to the selected remote service provider. It is checked whether the object region or symptom information of the image corresponds to an available specialty (39b) of the selected remote service provider by use of a specialty database component (25). If it is judged in authorization control that the object region or symptom information of the image does not correspond to the available specialty, distribution of the image is rejected.

## Description

### BACKGROUND OF THE INVENTION

### 1. Field of the Invention

The present invention relates to a remote diagnostic system and method for a medical image. More particularly, the present invention relates to a remote diagnostic system and method in which a remote service provider is requested by a service requester for image interpretation of a medical image, and an error in the transmission from the service requester to the remote service provider can be prevented.

### 2. Description Related to the Prior Art

In the field of medicine, various imaging modalities or medical examination apparatuses are widely used in hospital or other medical facilities. Examples of the imaging modalities include CR (computed radiography), DR (digital radiography), CT (computed tomography) and MRI (magnetic resonance imaging) apparatuses. Images are created in the examination, and viewed an diagnosed by a radiologist or specialist for image interpretation. A physician, doctor, surgeon, practitioner or the like having consulted a patient requests the radiologist to diagnose the images. A medical report of the image interpretation is made and supplied to the physician.

The Picture Archiving and Communication System (PACS) is installed in a large hospital for transmitting medical images, medical report, and other data by use of the network. The physician can receive images and the like with the PACS in connection with the radiologist.

In a health care provider (such as a small hospital or clinic) without a radiologist, an image cannot be diagnosed by the image interpretation in the facility even with the imaging modality. A request for the image interpretation must be placed to a large hospital with a radiologist as a remote service provider. JP-A 2001-243130 discloses a remote diagnostic system in which first and second terminal apparatuses are disposed in a service requester (health care provider) and the remote service provider, and coupled to one another communicably by a network service for the medical use. An image selected in the first terminal apparatus in the service requester is sent to the second terminal apparatus of one of the remote service providers, so as to place a request for the image interpretation.

Also, the DICOM (digital imaging and communications) is the widely used standards of file formats for medical images of digital form and communication technology. Files of the DICOM standards have an image area for storing data of the image, and an additional area for storing attribute information. The attribute information has patient information of a patient's name, patient ID, sex, age and the like. An accident is likely to occur in the transmission. If an unintended hospital is selected for transmitting images, personal information of the patient will be leaked. There is a suggestion in JP-A 2001-243130 in which personal information of patients is protected by information security of encrypting the medical image and medical data.

However, errors in the transmission of the images cannot be prevented in the remote diagnostic system disclosed in JP-A 2001-243130. Should images be transmitted erroneously even with high urgency or high importance, supply of a medical report of the image interpretation will be late, to cause serious influence to diagnosis, treatment and the like of the patient.

### SUMMARY OF THE INVENTION

In view of the foregoing problems, an object of the present invention is to provide a remote diagnostic system and method in which a remote service provider is requested by a service requester for image interpretation of a medical image, and an error in the transmission from the service requester to the remote service provider can be prevented.

In order to achieve the above and other objects and advantages of this invention, a remote diagnostic system includes an image distributor for requesting at least one remote service provider among plural remote service providers to diagnose a medical image, and transmitting the image thereto. An attribute input device inputs attribute information constituted by at least one of an object region and symptom information of an object in the image for a request of diagnosis. A remote service provider selector selects the one remote service provider among the plural remote service providers, to transmit the image and the attribute information to the selected remote service provider. A specialty database component stores specialty information constituted by plural specialties in relation to the diagnosis and an object region and symptom information associated with the specialties. An authorization control device checks by use of the specialty database component whether the object region or the symptom information of the image corresponds to an available specialty of the selected remote service provider, for, if it is judged that the object region or the symptom information of the image corresponds to the available specialty, authorizes the image distributor to distribute the image to the selected remote service provider, and for, if it is judged that the object region or the symptom information of the image does not correspond to the available specialty, rejects distribution of the image from the image distributor.

The authorization control device acquires a first specialty from the specialty database component in association with the object region or the symptom information input by the attribute input device, and checks whether the first specialty is included in the available specialty. The object region or the symptom information, if the first specialty is included in the available specialty, is judged to correspond to the available specialty, and if the first specialty is not included in the available specialty, is judged not to correspond to the available specialty.

Furthermore, a notifier operates if the authorization control device judges that the object region or the symptom information of the image does not correspond to the available specialty, and generates a rejection signal for notifying rejection of the distribution of the image.

The notifier further notifies name information of a second remote service provider with a specialty to which the object region or the symptom information of the image corresponds upon notification of the rejection.

Furthermore, a first terminal apparatus has the attribute input device and the remote service provider selector. The selected remote service provider has a second terminal apparatus for providing the first terminal apparatus with information of a medical report created by the diagnosis of the image.

The image is formed by use of one of imaging modalities of CR, DR, CT and MRI.

Also, a remote diagnostic method is provided, in which at least one remote service provider among plural remote service providers is requested to diagnose a medical image, and the image is transmitted thereto. Attribute information constituted by at least one of an object region and symptom information of an object in the image is input for a request of diagnosis. The one remote service provider is selected among the plural remote service providers, to transmit the image and the attribute information to the selected remote service provider. It is checked whether the object region or the symptom information of the image corresponds to an available specialty of the selected remote service provider, by use of a specialty database component for storing specialty information constituted by plural specialties in relation to the diagnosis and an object region and symptom information associated with the specialties. If it is judged that the object region or the symptom information of the image corresponds to the available specialty, distribution of the image to the selected remote service provider is authorized. If it is judged that the object region or the symptom information of the image does not correspond to the available specialty, distribution of the image is rejected.

In the checking step, a first specialty is acquired from the specialty database component in association with the object region or the symptom information input by the inputting step, and it is checked whether the first specialty is included in the available specialty. The object region or the symptom information, if the first specialty is included in the available specialty, is judged to correspond to the available specialty, and if the first specialty is not included in the available specialty, is judged not to correspond to the available specialty.

If it is judged that the object region or the symptom information of the image does not correspond to the available specialty, a rejection signal is generated for notifying rejection of the distribution of the image.

In addition to notifying the rejection, name information of a second remote service provider with a specialty to which the object region or the symptom information of the image corresponds is notified.

The remote service provider creates a medical report by the diagnosis of the image.

The image is formed by use of one of imaging modalities of CR, DR, CT and MRI.

Also, a service requesting system is provided, including an image distributor for requesting at least one remote service provider among plural remote service providers to diagnose a medical image, and transmitting the image thereto. An attribute input device inputs attribute information constituted by at least one of an object region and symptom information of an object in the image for a request of diagnosis. A remote service provider selector selects the one remote service provider among the plural remote service providers, to transmit the image and the attribute information to the selected remote service provider. A specialty database component stores specialty information constituted by plural specialties in relation to the diagnosis and an object region and symptom information associated with the specialties. An authorization control device checks by use of the specialty database component whether the object region or the symptom information of the image corresponds to an available specialty of the selected remote service provider, for, if it is judged that the object region or the symptom information of the image corresponds to the available specialty, authorizes the image distributor to distribute the image to the selected remote service provider, and for, if it is judged that the object region or the symptom information of the image does not correspond to the available specialty, rejects the image distributor from distributing the image.

Also, a service requesting method is provided, including a step of requesting at least one remote service provider among plural remote service providers to diagnose a medical image, and transmitting the image thereto. Attribute information constituted by at least one of an object region and symptom information of an object in the image is input for a request of diagnosis. The one remote service provider is selected among the plural remote service providers, to transmit the image and the attribute information to the selected remote service provider. It is checked whether the object region or the symptom information of the image corresponds to an available specialty of the selected remote service provider, by use of a specialty database component for storing specialty information constituted by plural specialties in relation to the diagnosis and an object region and symptom information associated with the specialties. If it is judged that the object region or the symptom information of the image corresponds to the available specialty, distribution of the image to the selected remote service provider is authorized. If it is judged that the object region or the symptom information of the image does not correspond to the available specialty, distribution of the image is rejected.

Consequently, an error in the transmission from the service requester to the remote service provider can be prevented, because availability of a specialty of a remote service provider is reliably checked in relation to an object region or symptom information of the image by use of a specialty database component.

### BRIEF DESCRIPTION OF THE DRAWINGS

The above objects and advantages of the present invention will become more apparent from the following detailed description when read in connection with the accompanying drawings, in which:
Fig. 1 is a block diagram schematically illustrating a remote diagnostic system;
Fig. 2 is a block diagram schematically illustrating a client terminal apparatus of a service requester;
Fig. 3 is a table illustrating a remote service provider database component;
Fig. 4 is a table illustrating a specialty database component;
Fig. 5 is a block diagram schematically illustrating circuit elements in a client terminal apparatus according to an application program;
Fig. 6 is an explanatory view in a front elevation illustrating a display panel with information displayed for requesting image interpretation;
Fig. 7 is an explanatory view illustrating a list of object regions and symptom information;
Fig. 8 is an explanatory view illustrating a list of remote service providers;
Fig. 9 is a block diagram schematically illustrating circuit elements in a distribution server according to an application program;
Fig. 10 is an explanatory view in a front elevation illustrating an example of an error massage;
Fig. 11 is a flow chart illustrating a flow of image interpretation;
Fig. 12 is an explanatory view in a front elevation illustrating another preferred error message.

### DETAILED DESCRIPTION OF THE PREFERRED EMBODIMENT(S) OF THE PRESENT INVENTION

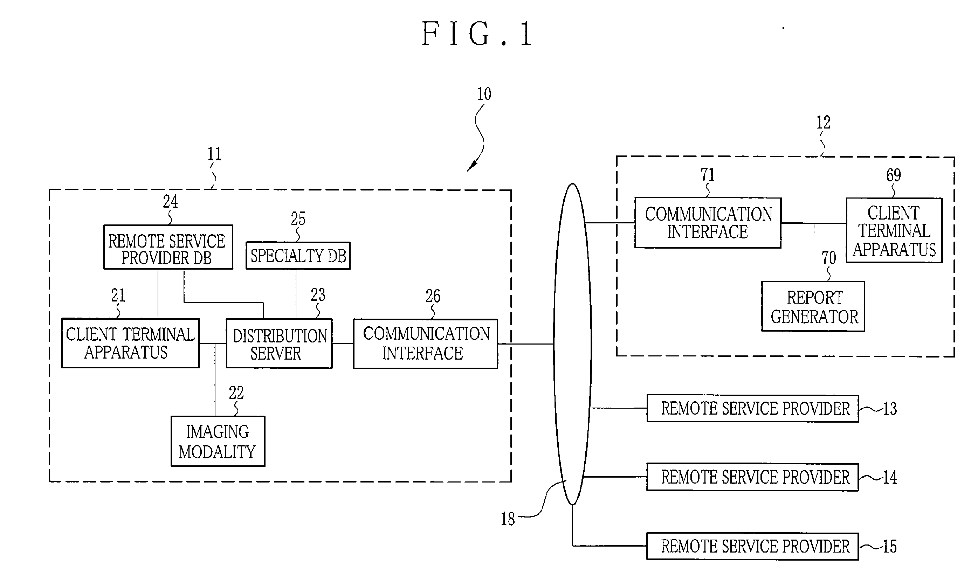

In Fig. 1, a remote diagnostic system 10 for radiology according to the invention includes at least one service requester 11 (health care provider) with a physician, doctor or practitioner, and a plurality of remote service providers 12, 13, 14 and 15 or service facilities for image interpretation. The service requester 11 is a facility for health care of a patient, for example, a hospital, clinic, health center and the like. No specialist for the image interpretation is present in the service requester 11. An image obtained by examining the patient is sent to and viewed in one remote service provider for diagnosis. Examples of the remote service providers 12-15 are a large scale hospital with radiologists, a medical center with a feature department of radiology, and the like. The remote service providers 12-15 are requested by the service requester 11 to view and interpret the image, creates a medical report and supplies the same to the service requester 11.

There is an IP network 18 for communicably coupling the remote service providers 12-15 to the service requester 11. A base network of the IP network 18 is a specialized large IP network provided by a communication service of the telecommunication industry. A virtual private network (VPN) is established on the large IP network to constitute the IP network 18 as a closed network. It is possible with the VPN to keep security protection in the IP network 18 without leakage of data to the outside of the remote diagnostic system 10.

The service requester 11 has a client terminal apparatus 21, an imaging modality 22, a distribution server 23 (in information security), a remote service provider database component 24, a specialty database component 25, and a communication interface 26. A local area network (LAN) is provided in the service requester 11, and interconnects the client terminal apparatus 21, the imaging modality 22, the distribution server 23 and the communication interface 26. The remote service provider database component 24 is connected to the client terminal apparatus 21 and the distribution server 23. The specialty database component 25 is connected to the distribution server 23.

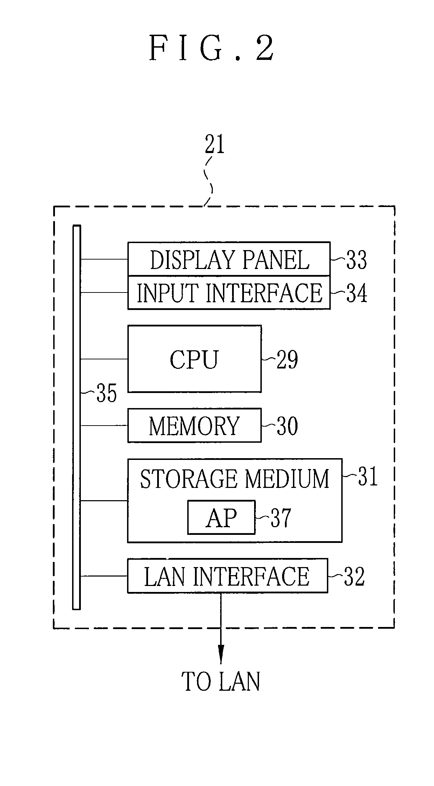

The client terminal apparatus 21, as a first terminal apparatus of the invention, is used for a request of image interpretation to the remote service providers 12-15, and for receiving a medical report from the remote service providers 12-15. An example of the client terminal apparatus 21 is a personal computer. In Fig. 2, the client terminal apparatus 21 includes a CPU 29, a memory 30, a storage medium 31 or storage device, a local area network interface 32 (LAN port), a display panel 33, and an input interface 34. Examples of the input interface 34 are a keyboard and mouse. A data bus 35 interconnects the CPU 29, the memory 30, the storage medium 31, the local area network interface 32, the display panel 33 and the input interface 34.

An example of the storage medium 31 is a hard disk drive (HDD). An application program (AP) 37 and a control program are installed in the storage medium 31. The application program 37 is for requesting the image interpretation. The memory 30 is a working memory with which the CPU 29 operates for processing. The CPU 29 reads the application program 37 and the like from the storage medium 31 to the memory 30, and runs the application program 37 to control various elements of the computer totally. The local area network interface 32 is an interface for transmission with the LAN in the service requester 11.

The imaging modality 22 is a medical apparatus for examination, for example, CR, DR, CT or MRI apparatus, for imaging of an object in a body of a patient. Medical images are created by the imaging modality 22, and transmitted by the LAN in the service requester 11 to the client terminal apparatus 21. The images are written to an image storage area in the storage medium 31. An example of the image storage area is one particular folder in the storage medium 31. The images are stored in a file format of the DICOM.

Note that the imaging modality 22 of the invention can be an ultrasound apparatus (ultrasonic echo apparatus) for ultrasonic imaging, an ECG machine for recording an electrocardiograph, and the like.

The distribution server 23 is used for distributing images to the remote service providers 12-15, and receiving a medical report from the remote service providers 12-15. The distribution server 23 is a personal computer constructed similarly to the client terminal apparatus 21, and includes a CPU, memory, storage medium, local area network interface, display panel, and input device such as a keyboard, mouse and the like. An application program (AP) is installed in the storage medium of the distribution server 23 for remote diagnosis in a manner similar to the client terminal apparatus 21. The CPU reads the application program from the storage medium, and runs the same by use of the memory.

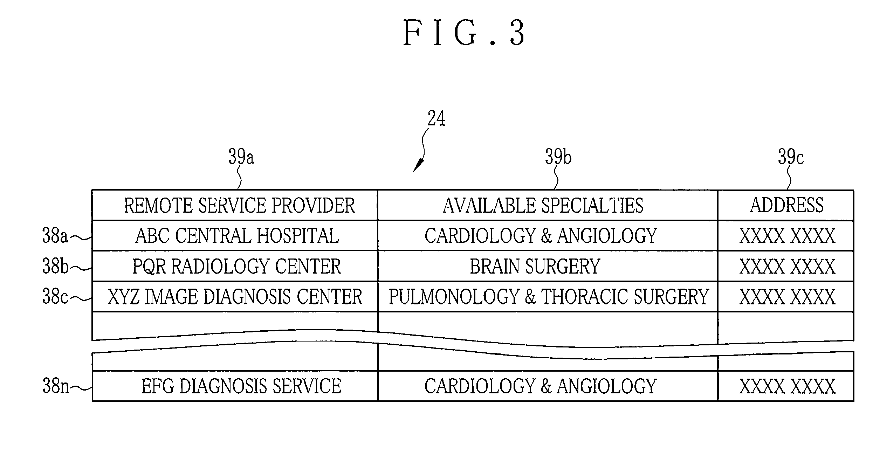

The remote service provider database component 24 is related to the remote service providers 12-15 with which image interpretation of images is requested by the service requester 11. In Fig. 3, the remote service provider database component 24 has remote service provider data 38a-38n of the remote service providers 12-15. Each of the remote service provider data 38a-38n is constituted by a plurality of fields, namely a remote service provider field 39a, an available specialty field 39b and an address field 39c. The remote service provider field 39a has a provider name. The available specialty field 39b has information of specialties, such as "cardiology and angiology", "brain surgery", "pulmonology and thoracic surgery", in which the remote service providers 12-15 are specialized. The address field 39c has address information of addresses to which images will be transmitted for the purpose of image interpretation. Examples of the address information are an IP address, mail address and the like.

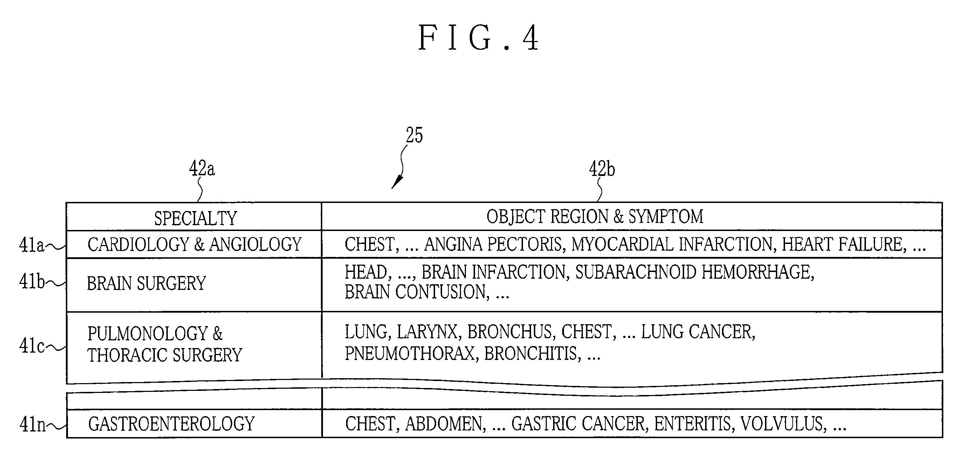

The specialty database component 25 contains information for defining contents of specialties recorded in the region of the "specialties of the remote service providers" in the remote service provider database component 24. As illustrated in Fig. 4, the specialty database component 25 contains a plurality of specialty data 41a-41n as datasets related to the specialties. Each of the specialty data 41a-41n is constituted by a specialty field 42a and an object/symptom field 42b. The object/symptom field 42b has information of an object region and symptom information of patients in relation to the particular specialty. For example, one of the items of the specialties is the "pulmonology and thoracic surgery". In relation to this, examples of the object regions are a lung, larynx, bronchus, chest and the like. Examples of symptom information are a lung cancer, pneumothorax, bronchitis and the like.

The communication interface 26 converts the data format according to a format for the LAN in the service requester 11 and a format for the IP network 18, to connect the LAN in the service requester 11 to the IP network 18. Examples of the communication interface 26 include a modem, router and the like, which can be selected suitably according to the standards of the LAN of the service requester 11 and the IP network 18.

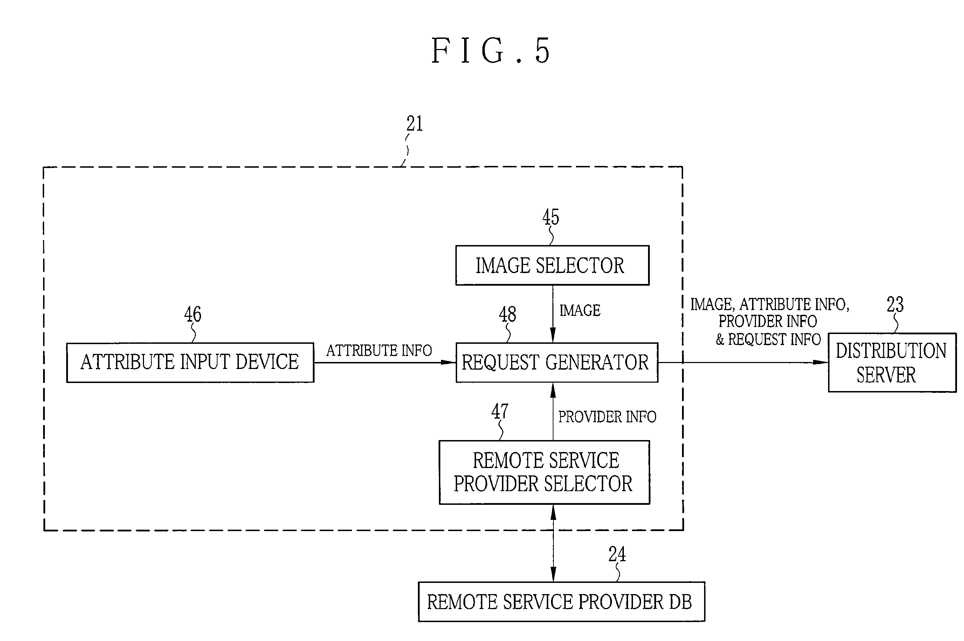

In the client terminal apparatus 21, the CPU 29 runs the application program 37. In Fig. 5, the client terminal apparatus 21 includes an image selector 45, an attribute input device 46, a remote service provider selector 47 and a request generator 48. The image selector 45 is caused to select an image for a request of image interpretation. The attribute input device 46 operates for an input of attribute information such as an object region and symptom information of an object of the selected image. The remote service provider selector 47 is caused to select one of the remote service providers 12-15 for the request of the image interpretation.

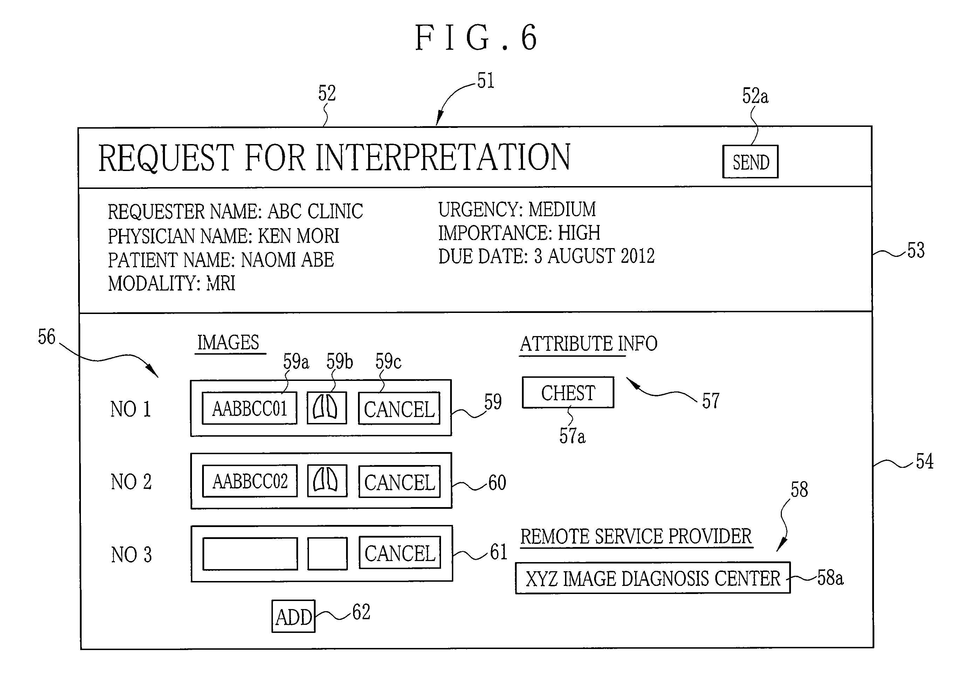

In Fig. 6, a service request form 51 is displayed on the display panel 33 of the client terminal apparatus 21 so that the image selector 45, the attribute input device 46 and the remote service provider selector 47 set data and command signal selectively according to inputs. The service request form 51 includes a title area 52, an information area 53 and a selection processing area 54. The title area 52 has a title of "REQUEST FOR INTERPRETATION", and also a send button 52a for transmission of an image. The send button 52a is disabled by the control of the service request form 51 before inputting an image, attribute information and a provider name of a remote service provider.

The information area 53 displays request information to be transmitted with the image in the course of the request for the image interpretation. The information area 53 indicates items including a requester name, physician name, patient name, imaging modality, urgency, importance and due date. The "requester name" is the name of the service requester 11, for example, ABC Clinic. The "physician name" is a name of a physician, doctor or practitioner requesting the image interpretation, and normally of a physician who cares for the patient in relation to the medical image. The "imaging modality" is selected from the DR, CR, CT, MRI and the like. Each of the "urgency" and "importance" is expressed with "low", "medium" or "high" as a level of the degree. The "due date" is a date before which the physician or doctor wishes to receive a medical report upon the request for the image interpretation. Note that it is possible for the physician or doctor to input the request information directly by use of the client terminal apparatus 21. Also, the request information can be created by reading attribute information from an electronic medical chart if a system of the electronic medical chart is utilized in the service requester 11.

The selection processing area 54 includes a selection field 56, an attribute field 57, and a remote service provider selection field 58. The selection field 56 is used for selecting images and is constituted by the image selector 45. The selection field 56 includes a first image field zone 59, a second image field zone 60 and a third image field zone 61. The first image field zone 59 includes a selection field area 59a or selection box, a thumbnail field area 59b or thumbnail box, and a cancel button 59c. The thumbnail field area 59b displays a thumbnail image of the selected image. The cancel button 59c is depressible for canceling selection of the image. When the selection field area 59a is clicked by the mouse of the input interface 34, images stored in the storage medium 31 are read and displayed in the listed form. The mouse is manipulated to select one of the displayed images, so that a file name of the selected image is indicated in the selection field area 59a.

The second and third image field zones 60 and 61 are similar to the first image field zone 59. An addition button 62 is depressed for selecting three or more images. An additional image field zone is displayed upon depression of the addition button 62 in a form similar to the image field zones 59-61.

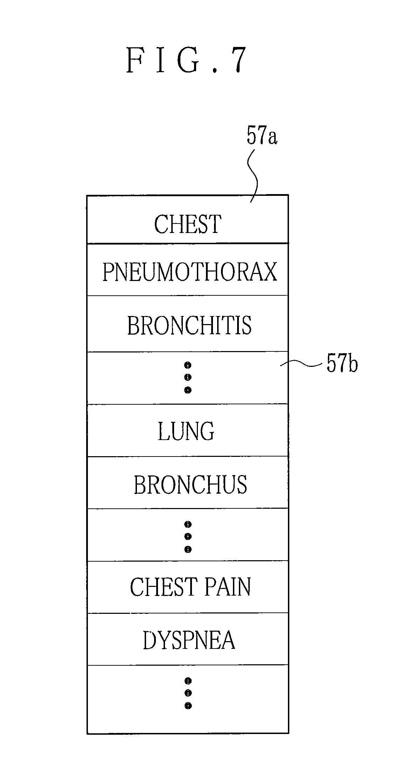

The attribute field 57 is used for inputting attribute information and is constituted by the attribute input device 46. The attribute field 57 has an information field area 57a (information box) for an object region and symptom information. When the information field area 57a is clicked by the mouse, a candidate list 57b or field region is displayed as illustrated in Fig. 7 for object regions and symptom information as candidate of attribute information. Information of the candidate list 57b is stored in the storage medium 31 or the like. When the operator selects one of the items in the candidate list 57b with the mouse, the selected item is indicated in the information field area 57a.

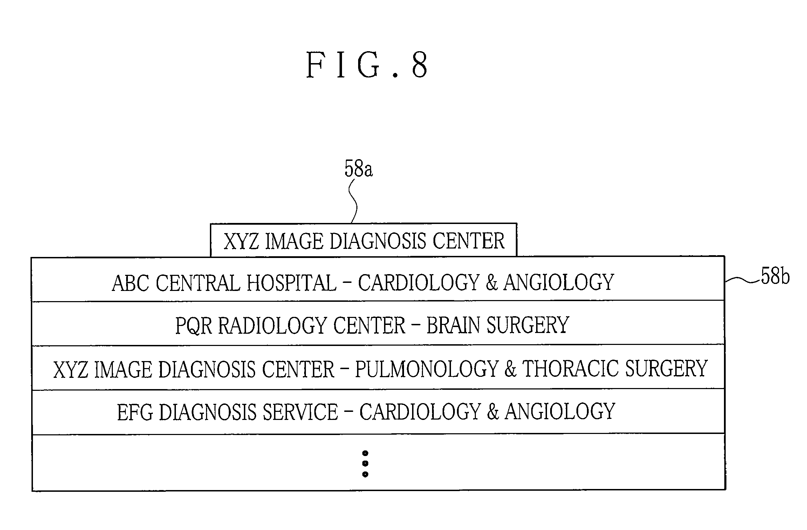

The remote service provider selection field 58 is used for selecting the remote service provider and is constituted by the remote service provider selector 47. A selection field area 58a for a remote service provider or selection box is disposed in the remote service provider selection field 58. When the selection field area 58a is clicked by use of the mouse, provider names and specialties of the remote service providers as candidates are read from the remote service provider database component 24 as illustrated in Fig. 8. A candidate list 58b or field region is indicated with the plural provider names and the plural specialties. A particular one of the provider names is selected in the candidate list 58b by the mouse, so that the selected provider name is indicated in the selection field area 58a.

The request generator 48 transmits the various data to the distribution server 23, the various data including the image selected by the image selector 45, the attribute information input by the attribute input device 46, information of the selected remote service provider from the remote service provider selector 47, and the request information described above.

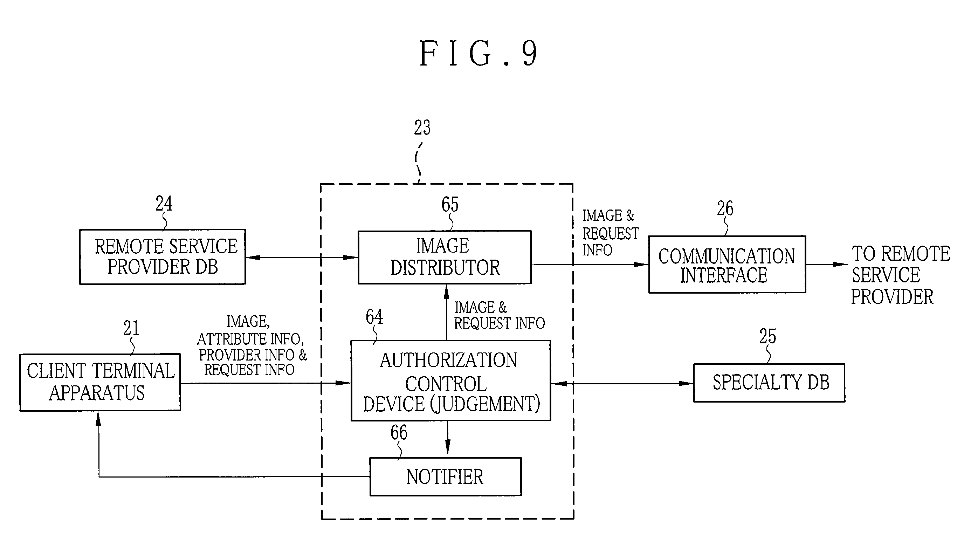

In the distribution server 23, the CPU runs the application program. In Fig. 9, the distribution server 23 includes an authorization control device 64, an image distributor 65 and a notifier 66. The distribution server 23 receives various data from the request generator 48 in the client terminal apparatus 21, such as the image, attribute information, information of the selected remote service provider, and request information.

The authorization control device 64 for information security checks whether the image selected in the image selector 45 is related to the available specialties of the remote service provider selected by the remote service provider selector 47. At first, the authorization control device 64 searches and acquires a first specialty from the specialty database component 25 according to the attribute information transmitted from the request generator 48. Then the authorization control device 64 accesses the remote service provider database component 24, reads the available specialties of the remote service provider selected by the remote service provider selector 47, and checks whether the available specialties coincide with (include) the first specialty acquired from the specialty database component 25. If the available specialties coincide with (include) the first specialty, the authorization control device 64 judges that the image is related to the available specialties of the remote service provider. If the available specialties do not coincide with (do not include) the first specialty, the authorization control device 64 judges that the image is not related to the available specialties of the remote service provider.

Let the "chest" be an object region as attribute information input by the attribute input device 46. Let "XYZ Image Diagnosis Center" in Fig. 3 be a remote service provider selected by the remote service provider selector 47. As illustrated in Fig. 4, specialties corresponding to the "chest" as object region information are the "cardiology and angiology", "pulmonology and thoracic surgery", and "gastroenterology". The specialty of the "pulmonology and thoracic surgery" of "XYZ Image Diagnosis Center" coincides with one of the specialties searched and acquired from the specialty database component 25. Then the authorization control device 64 judges that the image is related to the specialty of the remote service provider.

Let the "head" be the object region as attribute information input by the attribute input device 46. Let "XYZ Image Diagnosis Center" in Fig. 3 be the remote service provider selected by the remote service provider selector 47. In Fig. 4, the specialty related to the "head" is "brain surgery". The "pulmonology and thoracic surgery" as available specialties of "XYZ Image Diagnosis Center" does not coincide with the specialty acquired from the specialty database component 25. Thus, the authorization control device 64 judges that the image is not related to the available specialties of the selected remote service provider.

If the authorization control device 64 judges that the image is in the available specialties of the selected remote service provider selected by the remote service provider selector 47, the image distributor 65 is authorized to transmit the image and the request information to a client terminal apparatus 69 of the selected remote service provider. If the authorization control device 64 judges that the image is not in the available specialties of the remote service provider, the image distributor 65 is inhibited from outputting the image and the request information, because of the rejection. This is for the purpose of preventing leakage of personal information of the patient, because the images must not be transmitted to the remote service provider of available specialties different from that related to the image.

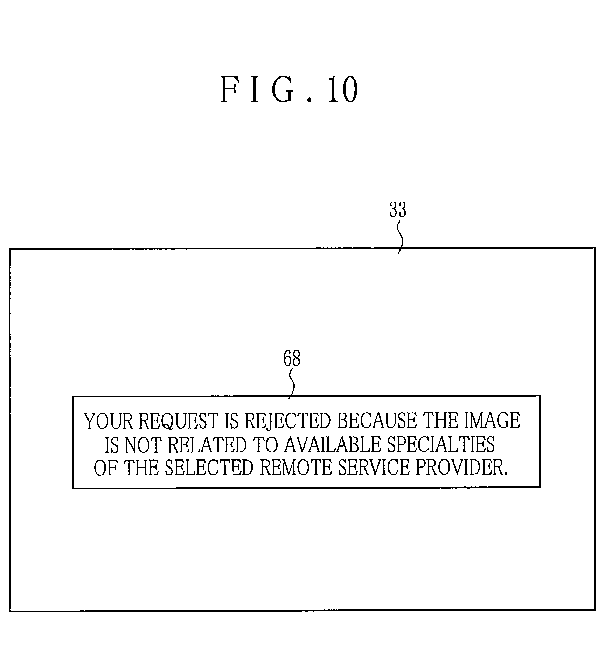

If the authorization control device 64 judges that the image is not related to the available specialties of the selected remote service provider, the notifier 66 notifies the client terminal apparatus 21 of the result of the judgement. In Fig. 10, an error message 68 is displayed on the display panel 33 of the client terminal apparatus 21 to inform rejection of the request of the image interpretation.

The remote service provider 12 has a report generator 70 or terminal apparatus, a communication interface 71 and the client terminal apparatus 69. There is a local area network (LAN) in the remote service provider 12 for interconnecting the client terminal apparatus 69, the report generator 70 and the communication interface 71. An example of the client terminal apparatus 69 is a personal computer in the same form as the client terminal apparatus 21 of the service requester 11. The client terminal apparatus 69, as a second terminal apparatus of the invention, receives a request for image interpretation from the service requester 11 and sends information of the medical report to the service requester 11. The communication interface 71 converts the data format according to a format for the LAN in the remote service provider 12 and a format for the IP network 18, to connect the LAN in the remote service provider 12 to the IP network 18. Examples of the communication interface 71 include a modem, router and the like in a manner similar to the communication interface 26 in the service requester 11.

An example of the report generator 70 is a personal computer similar to the client terminal apparatus 21. The report generator 70 operates for image interpretation of an image received by the client terminal apparatus 69, and for creating a medical report. The report generator 70 receives the image from the client terminal apparatus 69 upon receiving the request for image interpretation. The report generator 70 causes the display panel to display the image for a radiologist to view the image. Upon creating the medical report, the report generator 70 displays an entry field for creating the medical report on the display panel, and receives manual inputs of medical information from the input device such as a keyboard. The medical report created by the report generator 70 is transmitted to the client terminal apparatus 69. The remote service providers 13-15 are constructed equally to the remote service provider 12. Elements similar to those of the remote service provider 12 are designated with identical reference numerals.

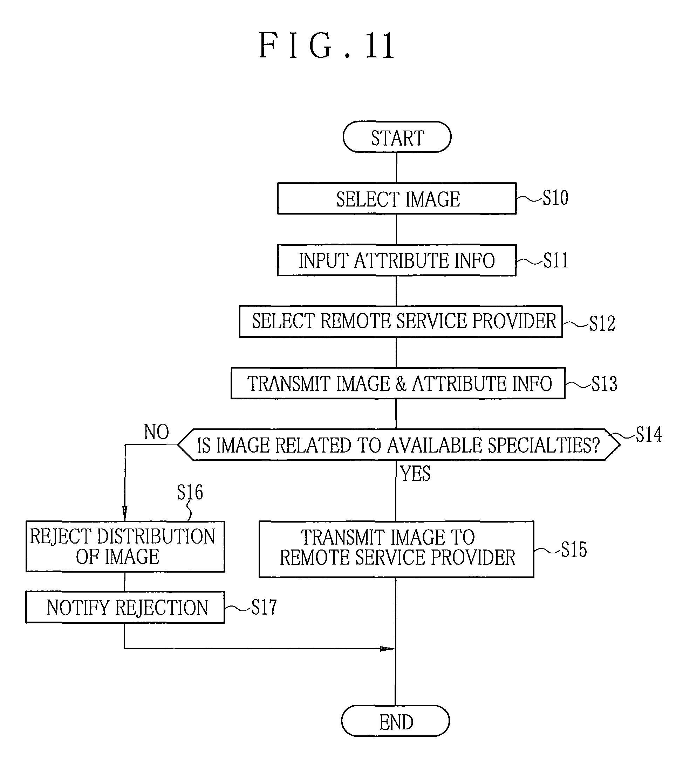

The operation of the above construction is described now by referring to a flow in Fig. 11. A physician, doctor or practitioner in the service requester 11 (health care provider) in Fig. 1 manipulates the client terminal apparatus 21 to run the application program 37 for a request of image interpretation to the remote service providers 12-15. In Fig. 5, the client terminal apparatus 21 operates with the image selector 45, the attribute input device 46, the remote service provider selector 47 and the request generator 48 upon running the application program 37. Also, an application program for image interpretation is run in the distribution server 23 in response to running of the application program 37 in the client terminal apparatus 21. The distribution server 23 operates with the authorization control device 64, the image distributor 65 and the notifier 66 of Fig. 9 upon running the application program for the image interpretation.

In Fig. 6, the display panel 33 of the client terminal apparatus 21 displays the service request form 51. A physician, doctor or practitioner of the service requester 11 manipulates the input interface 34 such as a keyboard and mouse for inputs in the service request form 51. In the selection field 56, one of the images related to a request of image interpretation is selected in the step S10. The attribute field 57 receives an input of attribute information which may be an object region and symptom information of an object of a body in the image in the step S11. In the remote service provider selection field 58, a remote service provider is selected for request of image interpretation of the image in the step S12. When the send button 52a of the service request form 51 is depressed, the request generator 48 causes the client terminal apparatus 21 to send the various data to the distribution server 23 in the step S13, the data including the image, the attribute information, provider name, request information displayed in the information area 53, and the like.

The authorization control device 64 of the distribution server 23 searches and acquires attribute information from the specialty database component 25, and checks whether the image from the client terminal apparatus 21 is related to the available specialties of the selected remote service provider in the step S14. If the authorization control device 64 judges that the image is related to the available specialties of the remote service provider (yes in the step S14), the image distributor 65 is authorized to transmit the image and the request information to that among the remote service providers 12-15 in the step S15.

If the authorization control device 64 judges that the image is not related to the available specialties of the remote service provider (no in the step S14), then the image distributor 65 is inhibited from transmission of the image to the remote service providers 12-15 in the step S16 by rejection, as no authorization is carried out. The notifier 66 sends the error message 68 of Fig. 10 to the client terminal apparatus 21 for the display panel 33 to display the error message 68. The physician, doctor or practitioner of the service requester 11 is notified of the rejection in the step S17.

In conclusion, the distribution of the images is rejected if an error has occurred in selecting the remote service provider for image interpretation. The information security can prevent transmission of an image to an unintended remote service provider, and avoid leakage of personal information of a patient. When the distribution of the images is rejected, the client terminal apparatus 21 is notified of the information of the rejection. The physician, doctor or practitioner can find the error in the selection of the remote service provider. He or she in the service requester 11 can properly restart a request for the image interpretation. No influence to diagnosis of the patient occurs because errors in the request are prevented.

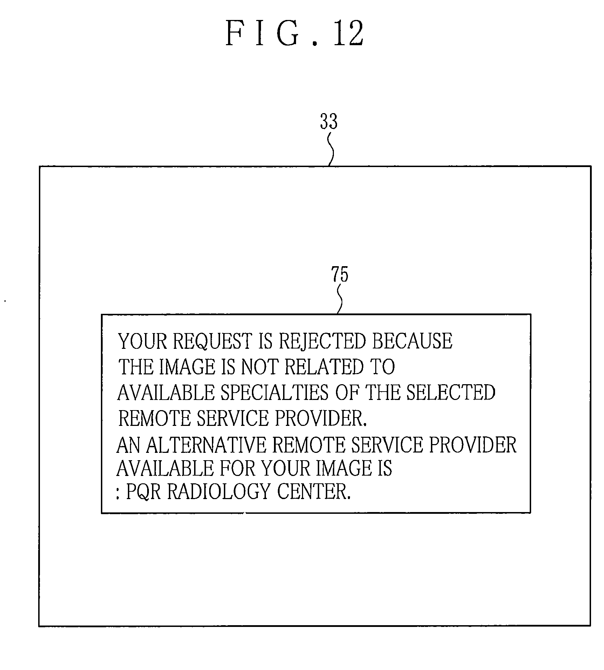

In the above embodiment, the client terminal apparatus 21 is notified of the rejection of the request for the image interpretation at the time of the rejection. Also, it is possible automatically to search and acquire information of an alternative remote service provider suitable for image interpretation of the image from the remote service provider database component 24, namely a remote service provider with available specialties to which the image corresponds. As illustrated in Fig. 12, an error message 75 can be displayed in the display panel 33 of the client terminal apparatus 21 with a name of the alternative remote service provider.

In the above embodiments, the remote service providers are medical facilities. However, a remote service provider is not limited to a facility, but can be in a small scale. A single person as a radiological specialist with the client terminal apparatus 69 can constitute a remote service provider. Also, the report generator 70 and the client terminal apparatus 69 can be combined as a single apparatus.

Furthermore, a system of the invention with the service requester and the remote service providers can be a combination of an outpatient department and radiology departments in a large hospital or other multiple medical facilities.

Also, it is possible in the invention to combine known techniques in the field of service requesting, information security and the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A remote diagnostic system including an image distributor (65) for requesting at least one remote service provider (58a) among plural remote service providers (12-15, 38a-38n, 58b) to diagnose a medical image (59b), and transmitting said image thereto, **characterized in** comprising:
an attribute input device (46) for inputting attribute information (57) constituted by at least one of an object region and symptom information (42b, 57b) of an object in said image for a request of diagnosis;
a remote service provider selector (47) for selecting said one remote service provider among said plural remote service providers, to transmit said image and said attribute information to said selected remote service provider;
a specialty database component (25) for storing specialty information (42b) constituted by plural specialties (41a-41n) in relation to said diagnosis and an object region and symptom information associated with said specialties;
an authorization control device (64) for checking by use of said specialty database component whether said object region or said symptom information of said image corresponds to an available specialty (39b) of said selected remote service provider, for, if it is judged that said object region or said symptom information of said image corresponds to said available specialty, authorizing said image distributor to distribute said image to said selected remote service provider, and for, if it is judged that said object region or said symptom information of said image does not correspond to said available specialty, rejecting distribution of said image from said image distributor.

2. A remote diagnostic system as defined in claim 1, characterized that said authorization control device acquires a first specialty from said specialty database component in association with said object region or said symptom information input by said attribute input device, and checks whether said first specialty is included in said available specialty;
wherein said object region or said symptom information, if said first specialty is included in said available specialty, is judged to correspond to said available specialty, and if said first specialty is not included in said available specialty, is judged not to correspond to said available specialty.

3. A remote diagnostic system as defined in claim 1 or 2, **characterized in** further comprising a notifier for operating if said authorization control device judges that said object region or said symptom information of said image does not correspond to said available specialty, and generating a rejection signal for notifying rejection of said distribution of said image.

4. A remote diagnostic system as defined in claim 3, characterized that said notifier further notifies name information of a second remote service provider with a specialty to which said object region or said symptom information of said image corresponds upon notification of said rejection.

5. A remote diagnostic system as defined in any one of claims 1-4, **characterized in** further comprising a first terminal apparatus having said attribute input device and said remote service provider selector;
wherein said selected remote service provider has a second terminal apparatus for providing said first terminal apparatus with information of a medical report created by said diagnosis of said image.

6. A remote diagnostic system as defined in any one of claims 1-5, characterized that said image is formed by use of one of imaging modalities of CR, DR, CT and MRI.

7. A remote diagnostic method in which at least one remote service provider (58a) among plural remote service providers (12-15, 38a-38n, 58b) is requested to diagnose a medical image (59b), and said image is transmitted thereto, **characterized in** comprising steps of:
inputting attribute information (57) constituted by at least one of an object region and symptom information (42b, 57b) of an object in said image for a request of diagnosis;
selecting said one remote service provider among said plural remote service providers, to transmit said image and said attribute information to said selected remote service provider;
checking whether said object region or said symptom information of said image corresponds to an available specialty (39b) of said selected remote service provider, by use of a specialty database component (25) for storing specialty information (42b) constituted by plural specialties (41a-41n) in relation to said diagnosis and an object region and symptom information associated with said specialties;
if it is judged that said object region or said symptom information of said image corresponds to said available specialty, authorizing distribution of said image to said selected remote service provider;
if it is judged that said object region or said symptom information of said image does not correspond to said available specialty, rejecting distribution of said image.

8. A remote diagnostic method as defined in claim 7, characterized that in said checking step, a first specialty is acquired from said specialty database component in association with said object region or said symptom information input by said inputting step, and it is checked whether said first specialty is included in said available specialty;
wherein said object region or said symptom information, if said first specialty is included in said available specialty, is judged to correspond to said available specialty, and if said first specialty is not included in said available specialty, is judged not to correspond to said available specialty.

9. A remote diagnostic method as defined in claim 7 or 8, characterized that if it is judged that said object region or said symptom information of said image does not correspond to said available specialty, a rejection signal is generated for notifying rejection of said distribution of said image.

10. A remote diagnostic method as defined in claim 8, characterized that in addition to notifying said rejection, name information of a second remote service provider with a specialty to which said object region or said symptom information of said image corresponds is notified.

11. A remote diagnostic method as defined in any one of claims 7-10, characterized that said remote service provider creates a medical report by said diagnosis of said image.

12. A remote diagnostic method as defined in any one of claims 7-11, characterized that said image is formed by use of one of imaging modalities of CR, DR, CT and MRI.
